# EUROPEAN PATENT APPLICATION

(11) **EP 2 236 626 A1**
(43) Date of publication of application: **06.10.2010**
(21) Application number: 08856538.7
(22) Date of filing: 04.12.2008
(51) Int. Cl.: C12Q 1/68, G01N 33/574

(54) **GENOMIC IMPRINTING FOR THE PROGNOSIS OF THE COURSE OF COLORECTAL ADENOCARCINOMA**

(30) Priority: 04.12.2007 ES 200703229
(71) Applicant: Universidad Autónoma De Madrid, 28049 Madrid (ES)
(72) Inventor: CASADO SÁENZ, Enrique, 28702 San Sebastián De Los Reyes - Madrid (ES); CEJAS GUERRERO, Paloma, E-28046 Madrid (ES); SÁNCHEZ HERNÁNDEZ, José Javier, E-28049 Madrid (ES); GONZÁLEZ BARÓN, Manuel, E-28046 Madrid (ES); FELIU BATTLE, Jaime, E-28046 Madrid (ES); DE CASTRO CARPENO, Javier, E-28046 Madrid (ES); BELDA INIESTA, Cristobal, E-28046 Madrid (ES)
(74) Representative: ABG Patentes, S.L.
(86) International application number: PCT/ES2008/000756
(87) International publication number: WO 2009/071720

(57) **Abstract**

The present invention relates to *in vitro* methods for establishing a prognosis concerning the progress of an individual diagnosed with colorectal cancer after the administration of a therapy, monitoring the effect of said therapy on the individual or predicting the clinical response of said individual to the therapy with respect to basal expression levels. Said methods comprise quantifying the expression level of the *TFF3* gene or of the protein encoded by said gene.

## Description

### Field of the Invention

The present invention relates to *in vitro* methods for predicting the clinical response of an individual diagnosed with colorectal adenocarcinoma to a therapy, monitoring the effect of the therapy administered to said individual and designing a customized therapy for an individual diagnosed with colorectal adenocarcinoma.

### Background of the Invention

Large intestine cancer is the third most common malignant tumor in the Western world, following lung and breast cancer, representing 10% of all cancers and causing 10% of cancer-related deaths. Approximately a third of all large intestine cancers are formed in the rectum, and most of these cancers are diagnosed as locally advanced rectal cancers (LARCs).

In LARC, the development of the Total Mesorectal Excision (TME) and of preoperative strategies with chemotherapy and radiotherapy has considerably improved overall survival, local control and probably the proportion of procedures which preserve the anal sphincter. Furthermore, chemoradiotherapy (CRT) toxicity has thus been mitigated.

According to the data from old and recent randomized clinical trials, the combined preoperative treatment with CRT has extended overall 5 year survival from 45% with conventional surgery (Douglass, H.O., et al. 1986, N.Engl.J Med., 315: 1294-1295; Tveit, K.M., et al. 1997 Norwegian Adjuvant Rectal Cancer Project Group, Br.J Surg., 84: 1130-1135), to 66-76% with preoperative CRT, and local recurrences have decreased from 30-50% to 6-8%. However, a third of the patients will develop distant metastases, which are responsible for the elevated mortality (Sauer, R., et al. 2004 N.Engl.J.Med., 351: 1731-1740; Bosset, J.F., et al. 2006 N.Engl.J Med., 355: 1114-1123).

Furthermore, a significant number of patients suffer considerable adverse effects associated with CRT with no benefits whatsoever. In contrast, other patients can be under-treated. It is therefore very interesting to identify the potential targets involved in the resistance to CRT and to identify the patients who will relapse and would benefit from more intensive treatments.

The selection of the CRT treatment for each individual case of rectal adenocarcinoma depends on clinical and pathological variables which are unable to predict the therapeutic efficacy. Individual targeted strategies which correlate specific basal biomarkers with the response to the preoperative CRT treatment have shown markers of interest, including the mutations in p53 (Kandioler, D. et al., 2002. Ann.Surg., 235:493-498; Rebischung, C., et al. 2002, Int. J Cancer, 100: 131-135), expression of thymidylate synthase (Johnston, P.G., et al. 1994, J Clin Oncol, 12: 2640-2647; Edler, D. et al., 2000, Clin Cancer Res. 6:1378-1384), p21 (Reerink, O., et al. 2004, Anticancer Res., 24:1217-1221; Qiu, H., et al. 2000, Dis. Colon Rectum, 43: 451-459; Fu, C.G., et al. 1998, Dis. Colon Rectum, 41: 68-74; Rau, B., et al. 2003, J Clin Oncol, 21: 3391-3401), EGFR (Milas, L., et al. 2004, Int. J Radiat.Oncol Biol.Phys., 58: 966-971), COX 2 (Smith, F.M., et al. 2006 Int.J.Radiat.Oncol.Biol.Phys., 64: 466-472), rate of spontaneous apoptosis (Rodel, C., et al. 2002, Int. J Radiat.Oncol Biol.Phys., 52: 294-303; Abe, T., et al. 2001, Anticancer Res., 21: 2115-2120) or CEA (Park, Y.A., et al. 2006, J Surg.Oncol, 93: 145-150). However, none of them has clearly demonstrated their predictive usefulness in clinical practice.

Since exposure to CRT induces the clonal expansion of resistant cancer cells, the dynamic evaluation of markers which respond to CRT has sparked enormous interest in rectal cancer (Crane, C.H., et al. 2003, J Clin Oncol, 21: 3381-3382). Blind gene screening through this strategy can identify new prognostic and potentially therapeutic targets. Furthermore, comparisons between patients reduce the sources of variability, offering an attractive context for target screening.

Few clinical studies have approached the dynamic evaluation of individual markers before and after treatment. Rau *et al*. found in a series of 66 patients that the reduction in the immunohistochemical expression of p21, and the increase in the expression of Ki-67, was associated with a worse prognosis (Rau, B., et al. 2003, J Clin Oncol, 21: 3391-3401). Similarly, in 40 patients included in the mentioned German trial, the persistence of lymph node disease after treatment and the increase in the expression of thymidylate synthase therein, after laser microdissection of the tumor, was accompanied by a higher rate of recurrences (Liersch, T. et al. 2006, J Clin Oncol, 24: 4062-4068).

International patent application WO2006/037993, of Auvation Limited, describes markers of cancer, such as ovarian and colon cancers, and uses of these as prognostic and diagnostic markers. The markers described correspond to cytochrome p450 enzymes: CYP51, CYP52, CYP2U1, CYP3A43, CYP4F11, CYP4X1, CYP4Z1, CYP26A1, CYP3A7, CYP2F1, CYP4V2 and CYP39.

International patent application WO2007/073220, of Pacific Edge Biotechnology Limited, describes compositions and methods for determining the prognosis of cancer in a patient, particularly, colon cancer. Specifically, this invention relates to the use of genetic markers for the prediction of the prognosis of cancer based on a characteristic signature (the genetic markers are described in Tables 1 and 2). The gene imprints obtained are shown in Tables 8a, 8b and table 9 of said document.

Barrier, A., et al., 2006 (Journal of Clinical Oncology, Vol.24(29): 4685-4691) describe the ability of a gene expression microarray to predict the prognosis of patients with stage II colon cancer. The evaluated microarrays are made up of 30 and 23 genes.

Knoesel, T., et al., 2005 (Neoplasia, Vol. 7(8): 741-747) describe the immunogenic profiles of 11 biomarkers for rectal cancer by means of tissue microarrays. The genes considered are Adam 10, CyclinD1, AnnexinII, NFBK, casein-kinase-2-beta (CK2B), YB-1, P32, Rad51, c-fos, IGFBP4, and connexin 26 (Cx26).

Finally, Watanabe, T., et al., 2006 (Cancer Res, Vol. 66(7): 3370-3374) describe a gene expression pattern useful for predicting the sensitivity of rectal cancer cells in response to pre-operative radiotherapy.

Therefore, in the state of the art there is a need to provide a new method which allows establishing the prognosis for the progress of an individual diagnosed with colorectal adenocarcinoma prior to the administration of a therapy once said individual has been subjected to therapy, and to thus identify which individuals will relapse after the therapy and which could therefore be object of an alternative treatment with respect to the one originally administered.

### Summary of the Invention

In one aspect, the present invention relates to an *in vitro* method for predicting the clinical response of an individual diagnosed with colorectal adenocarcinoma to a therapy, comprising
(i) quantifying the expression levels of the genes shown in Tables 1A and 1B, or
(ii) quantifying the levels of proteins encoded by the genes of Tables 1A and 1B, or any functionally equivalent variant of said proteins,
   in a biological sample from said individual before the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of a better response of the individual after the administration of the therapy.

In another aspect, the invention relates to an *in vitro* method for predicting the clinical response of an individual diagnosed with colorectal adenocarcinoma to the therapy, comprising
(i) quantifying the expression levels of the genes shown in Tables 2A and 2B, or
(ii) quantifying the levels of proteins encoded by the genes of Tables 2A and 2B, or any functionally equivalent variant of said proteins,
   in a biological sample from said individual before the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of a worse response of the individual after the administration of the therapy.

In another aspect, the invention relates to an *in vitro* method for monitoring the effect of the therapy administered to an individual diagnosed with colorectal adenocarcinoma, comprising
(i) quantifying the expression levels of the genes shown in Tables 1A and 1B, or
(ii) quantifying the levels of proteins encoded by the genes of Tables 1A and 1B, or any functionally equivalent variant of said proteins,
   in a biological sample from said individual after the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of good tumor response to the therapy.

In another aspect, the invention relates to an *in vitro* method for monitoring the effect of the therapy administered to an individual diagnosed with colorectal adenocarcinoma, comprising
(i) quantifying the expression levels of the genes shown in Tables 2A and 2B, or
(ii) quantifying the levels of proteins encoded by the genes of Tables 2A and 2B, or any functionally equivalent variant of said proteins,
   in a biological sample from said individual after the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of poor tumor response to the therapy.

In another aspect, the invention relates to an *in vitro* method for designing a customized therapy for an individual diagnosed with colorectal adenocarcinoma, comprising
(i) quantifying the expression levels of the genes shown in Tables 2A and 2B, or
(ii) quantifying the levels of proteins encoded by the genes of Tables 2A and 2B, or any functionally equivalent variant of said proteins,
in a biological sample from said individual, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative that the individual needs an alternative therapy with respect to standard therapy.

In another aspect, the invention relates to a reagent capable of detecting the expression levels of the genes of Tables 1 and 2 or the proteins encoded by said genes.

In another aspect, the invention relates to a DNA or RNA array comprising a set of nucleic acids, wherein said set of nucleic acids consists of the nucleic acid sequences of the genes of Tables 1 and 2, or a fragment thereof, or the products of their transcription.

Finally, in another aspect, the invention also relates to an array comprising a set of antibodies, wherein said set of antibodies consists of antibodies, or fragments thereof, which are able to bind specifically with the proteins encoded by the genes of Tables 1 and 2 or any functionally equivalent variant of said proteins.

### Brief Description of the Drawings

Figure 1 shows two graphs showing the relationship between tumor response and disease-free survival. Comparing the no response (NR) to the pathological response (TRG4), the median is not reached in the time to relapse, but the value of p=0.05 is significant (A). When it is analyzed by subgroups (B), by adding the good response (GR, TRG 2/3), the same tendency is observed, without reaching statistical significance.
Figure 2 is a diagram showing the analysis of the signaling established between the members of the genomic imprinting generated from tumor response.

### Detailed Description of the Invention

The selection of the chemotherapy treatment for each individual case of colorectal adenocarcinoma depends on clinical and pathological variables which are unable to predict the therapeutic efficacy of a treatment or to establish a prognosis concerning the progress of a patient suffering said type of cancer. The inventors have identified a set of 13 genes the quantification of the expression of which with respect to constitutive genes surprisingly allows (i) predicting the better or worse progress of an individual diagnosed with colorectal adenocarcinoma after the administration of a therapy; (ii) monitoring the effect of the therapy administered to an individual diagnosed with colorectal adenocarcinoma and establishing a prognosis as to if the individual will respond well to it; and (iii) designing a customized therapy for an individual diagnosed with colorectal adenocarcinoma, deciding the type of therapy suited for each individual.

| | |
|---|---|
| **Table 1 Prediction of response and/or good progress of the patient** | |

| **A. Expressed more** | **B. Expressed less** |
|---|---|
| ALDH1A1 | BAK1 |
| MLH1 | CDKN1 |
| RELB | FOS |
| TYMS | STAT3 |
| GPX2 | CKB |
| *TFF3* | HIG2 |
| | PH-4 |
| **Table 2 Prediction of no response and/or poor progress of the patient** | |

| **A. Expressed more** | **B. Expressed less** |
|---|---|
| BAK1 | ALDH1A1 |
| MLH1 | CDKN1 |
| RELB | FOS |
| TYMS | STAT3 |
| CKB | GPX2 |
| *TFF3* | HIG2 |
| PH-4 | |

Therefore, in one aspect, the invention relates to an *in vitro* method for predicting the clinical response of an individual diagnosed with colorectal adenocarcinoma to a therapy comprising
(i) quantifying the expression levels of the genes shown in Tables 1A and 1B, or
(ii) quantifying the levels of proteins encoded by the genes of Tables 1A and 1B, or any functionally equivalent variant of said proteins,
   in a biological sample from said individual before the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of a better response of the individual after the administration of the therapy.

In another aspect, the invention relates to an *in vitro* method for predicting the clinical response of an individual diagnosed with colorectal adenocarcinoma to the therapy, comprising
(i) quantifying the expression levels of the genes shown in Tables 2A and 2B, or
(ii) quantifying the levels of proteins encoded by the genes of Tables 2A and 2B, or any functionally equivalent variant of said proteins,
in a biological sample from said individual before the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of worse response of the individual after the administration of the therapy.

In a particular embodiment, the parameter indicating the progress of an individual diagnosed with colorectal adenocarcinoma before the administration of the therapy is selected from the group of tumor response, risk of relapse, the disease-free survival and/or overall survival of the individual.

In the present invention, "risk of relapse" is understood as the probability of an individual to re-develop colorectal adenocarcinoma after a disease-free period.

In the present invention, "disease-free survival" is understood as time period after treatment in which the cancer is not found.

In the present invention, "overall survival of the individual" is understood as the percentage of individuals who survive from the time of the diagnosis or treatment until the end of a defined time period.

In another aspect, the invention relates to an *in vitro* method for monitoring the effect of the therapy administered to an individual diagnosed with colorectal adenocarcinoma, comprising
(i) quantifying the expression levels of the genes shown in Tables 1A and 1B, or
(ii) quantifying the levels of proteins encoded by the genes of Tables 1A and 1B, or any functionally equivalent variant of said proteins,
in a biological sample from said individual after the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of good tumor response to the therapy.

In another aspect, the invention relates to an *in vitro* method for monitoring the effect of the therapy administered to an individual diagnosed with colorectal adenocarcinoma, comprising
(i) quantifying the expression levels of the genes shown in Tables 2A and 2B, or
(ii) quantifying the levels of proteins encoded by the genes of Tables 2A and 2B, or any functionally equivalent variant of said proteins,
   in a biological sample from said individual after the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of poor tumor response to the therapy.

In another aspect, the invention relates to an *in vitro* method for designing a customized therapy for an individual diagnosed with colorectal adenocarcinoma, comprising
(i) quantifying the expression levels of the genes shown in Tables 2A and 2B, or
(ii) quantifying the levels of proteins encoded by the genes of Tables 2A and 2B, or any functionally equivalent variant of said proteins,
in a biological sample from said individual, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative that the individual needs an alternative therapy with respect to standard therapy.

In the present invention, "alternative therapy" is understood as a therapy that is different from the one originally administered, herein referred to as standard therapy or conventional therapy, to an individual. Said "alternative therapy" includes significant variations to the standard therapy, such as the substitution of some agents with others, the addition of alternative chemotherapy agents, change of doses or increase of the dose intensity of the drugs, addition of other anti-cancer agents (approved or in the experimental phase), alteration in the administration sequence of the anti-cancer agents or the type of local treatments, such as surgery or radiotherapy, etc. The alternative therapies herein defined are probably associated with more side effects for the individual (though not necessarily) and foreseeably greater effectiveness. Specific examples of anti-cancer agents included within the alternative therapy could be irinotecan, bevacizumab and cetuximab, agents which attack signaling pathways, antiangiogenic agents, etc.

In the present invention, "standard therapy" or "conventional therapy" is understood as any therapy that uses the drugs with clinical efficacy proven in randomized phase III studies, alone or in combinations similar to those used in the present invention, for example, in colorectal cancer the use of oxaliplatin, 5-fluorouracil or oral fluoropyrimidines, irinotecan, bevacizumab, or cetuximab, in regimens such as FOLFOX, FOLFIRI, XELOX, or XELIRI, with or without bevacizumab, or cetuximab, etc.

As it is used herein, the term "protein" relates to a molecular chain of amino acids, bound by covalent or noncovalent bonds. The term furthermore includes all the forms of physiologically relevant post-translational chemical modifications, for example, glycosylation, phosphorylation or acetylation, etc., provided that the functionality of the protein is maintained.

In the present invention, "functionally equivalent variant" is understood as a protein the amino acid sequence of which (i) is substantially homologous to the amino acid sequence of a specific protein and (ii) performs the same functions as said specific protein. The functional similarity of one protein with another specific protein can be determined by means of interference assays with the expression of the gene encoding the specific protein which, upon reducing the expression, would reduce the activity of that protein and the subsequent recovery of the activity by means of expression of the sequence of the other protein. These experiments will be performed using specific interference RNA sequences complementary for the sequence of the specific protein and expression vectors incorporating the specific sequence of the other protein regulated by an inducible or non-inducible promoter.

An amino acid sequence is substantially homologous to a determined amino acid sequence when it presents a degree of identify of at least 70%, advantageously of at least 75%, typically of at least 80%, preferably of at least 85%, more preferably of at least 90%, still more preferably of at least 95%, 97%, 98% or 99%, with respect to said determined amino acid sequence. The degree of identity between two amino acid sequences can be determined by conventional methods, for example, by means of standard sequence alignment algorithms known in the state of the art, such as, for example, BLAST [Altschul S.F. et al. Basic local alignment search tool. J Mol Biol. 1990 Oct 5; 215(3):403-10].

In the present invention, "genes which are constitutively expressed" or "constitutively expressed genes" is understood as those genes which are always active or which are constantly being transcribed. Examples of genes which are constitutively expressed are 2-myoglobin, ubiquitin, 18S ribosomal protein, cyclophilin A, transferrin receptor, actin, GAPDH, tyrosine 3-monooxygenase/tryptophan 5-monooxygenase activation protein (YWHAZ), ubiquitin, beta-actin and β-2-microglobulin.

The person skilled in the art will understand that the mutations in the nucleotide sequence of the genes which give rise to conservative substitutions of amino acids in noncritical positions for the functionality of the protein, are evolutionally neutral mutations which do not affect its overall structure or its functionality. Said variants fall within the scope of the present invention. Also included within the scope of the invention are those functionally equivalent variants of the proteins described in the present invention presenting insertions, deletions or modifications of one or more amino acids with respect to the starting proteins, and furthermore conserving the same functions as said starting proteins.

Therefore, as it is used herein, the term "functionally equivalent variant" also includes any functionally equivalent fragment of one of the proteins described in the present invention. The term "fragment" relates to a peptide comprising a part of a protein. In this case, a functionally equivalent fragment of the proteins described in the present invention is a peptide or protein comprising a part of said proteins and the same functions as said proteins.

The expression levels of the genes of Tables 1 and 2 can be quantified based on the RNA resulting from the transcription of said genes (mRNA) or, alternatively, based on the complementary DNA (cDNA) of said genes. Therefore, in a particular embodiment, quantifying the expression levels of the genes of Tables 1 and 2 comprises quantifying the messenger RNA (mRNA) of the genes of Tables 1 and 2, or a fragment of said mRNA, the complementary DNA (cDNA) of the genes of said Tables 1 and 2, or a fragment of said cDNA, or mixtures thereof.

Additionally, the method of the invention can include performing an extraction step for the purpose of obtaining the total RNA, which can be done by means of conventional techniques (Chomczynski et al., Anal. Biochem., 1987, 162:156; Chomczynski P., Biotechniques, 1993, 15:532).

Virtually any conventional method can be used within the framework of the invention for detecting and quantifying the levels of mRNA encoded by the genes of Tables 1 and 2 or of its corresponding cDNA. By way of non-limiting illustration, the levels of mRNA encoded by said genes can be quantified by means of the use of conventional methods, for example, methods comprising mRNA amplification and quantifying the product of said mRNA amplification, such as electrophoresis and staining, or alternatively by means of Southern blot and the use of suitable probes, Northern blot and the use of specific probes of the mRNA of the genes of interest or of its corresponding cDNA, mapping with S1 nuclease, RT-LCR, hybridization, microarrays, etc., preferably by means of real time quantitative PCR using suitable sets of probes and primers. Similarly, the levels of the cDNA corresponding to said mRNA encoded by the genes of Tables 1 and 2 can also be quantified by means of the use of conventional techniques; in this case, the method of the invention includes a step of synthesis of the corresponding cDNA by means of reverse transcription (RT) of the corresponding mRNA followed by amplification and quantification of the product of said cDNA amplification. Conventional methods for quantifying the expression levels can be found, for example, in Sambrook et al., 2001. "Molecular cloning: a Laboratory Manual", 3rd ed., Cold Spring Harbor Laboratory Press, N.Y., Vol. 1-3.

In a particular embodiment of the invention, the expression levels of the genes of Tables 1 and 2 are quantified by means of a multiplex quantitative polymerase chain reaction (PCR) or a DNA or RNA array.

In addition, in order to put the invention into practice, besides quantifying the expression levels of the genes of Tables 1 and 2, the expression levels of the protein encoded by said genes, i.e., the proteins ALDH1A1, MLH1, RELB, TYMS, GPX2, *TFF3,* BAK1, CDKN1, FOS, STAT3, CKB, HIG2 and PH-4, can also be quantified. Thus, in a particular embodiment, quantifying the levels of the protein encoded by the genes of Tables 1 and 2 comprises quantifying the proteins encoded by the genes of said Tables 1 and 2.

The expression level of the proteins encoded by the genes of Tables 1 and 2 can be quantified by means of any conventional method which allows detecting and quantifying said proteins in a sample from an individual. By way of non-limiting illustration, the levels of said proteins can be quantified, for example, by means of the use of antibodies with the ability to bind to said proteins (or to fragments thereof containing an antigenic determinant) and subsequently quantifying the complexes formed. The antibodies which are used in these assays can be labeled or not. Illustrative examples of markers which can be used include radioactive isotopes, enzymes, fluorophores, chemiluminescent reagents, enzymatic substrates or cofactors, enzymatic inhibitors, particles, dyes, etc. There is a wide variety of known assays which can be used in the present invention which use non-labeled antibodies (primary antibody) and labeled antibodies (secondary antibody); these techniques include Western blot, ELISA (enzyme-linked immunosorbent assay), RIA (radioimmunoassay), competitive EIA (competitive enzymatic immunoassay), DAS-ELISA (double antibody sandwich ELISA), immunocytochemical and immunohistochemical techniques, techniques based on the use of protein biochips or microarrays including specific antibodies or assays based on colloidal precipitation in formats such as dipsticks. Other ways for detecting and quantifying said TFF3 protein include affinity chromatography techniques, ligand binding assays, etc. There are commercial antibodies for the proteins encoded by the genes shown in Tables 1 and 2 available on the market.

In a particular embodiment, the levels of protein encoded by the genes of Tables 1 and 2 are quantified by means of Western blot, ELISA, immunohistochemistry or a protein array.

In therapy for colorectal adenocarcinoma, a variety of treatments can be used for treating or eliminating or containing the cancer. Depending on the patient's health, as well as on the size, site and stage of the cancer, (i) surgery, consisting of eliminating the part of the colon or rectum containing cancer as well as some of the surrounding healthy tissue, (ii) cytotoxic/cytostatic treatments, such as chemotherapy, using anti-cancer drugs to destroy the cancer cells by circulating the drugs throughout the body through the blood stream; radiotherapy, in which high-energy radiations are used to kill the cancer cells, and anti-tumor agents; and/or (iii) immunotherapy, in which the administered compound stimulates, enhances or repairs the natural function of the immunological system against cancer to recognize and eliminate the cancer cells from the body, can be used.

Therefore, in a particular embodiment, the administered therapy is a treatment cytotoxic and/ or cytostatic which, in yet another more particular embodiment, said treatment is chemotherapy, radiotherapy, antitumor agents or combinations thereof.

Suitable chemotherapy agents include but are not limited to alkylating agents such as, for example, cyclophosphamide, carmustine, daunorubicin, mechlorethamine, chlorambucil, nimustine, melphalan and the like; anthracyclines, such as, for example, daunorubicin, doxorubicin, epirubicin, idarubicin, mitoxantrone, valrubicin and the like; taxane compounds, such as, for example, paclitaxel, docetaxel and the like; topoisomerase inhibitors such as, for example, etoposide, teniposide, irinotecan, tuliposide and the like; nucleotide analogues such as, for example, azacitidine, azathioprine, capecitabine, cytarabine, doxifluridine, fluorouracil, gemcitabine, mercaptopurine, methotrexate, thioguanine ftorafur and the like; platin-based agents such as, for example, carboplatin, cisplatin, oxaliplatin and the like; antineoplastic agents such as, for example, vincristine, leucovorin, lomustine, procarbazine and the like; hormonal modulators such as, for example, tamoxifen, finasteride, 5-α-reductase inhibitors and the like; vinca alkaloids such as, for example, vinblastine, vincristine, vindesine, vinorelbine and the like. The suitable chemotherapy agents are described in greater detail in the literature, such as in The Merck Index on CD-ROM, 13^{th} edition.

In a particular embodiment, chemotherapy comprises a compound selected from an alkylating agent preventing replication, a fluoropyrimidine, a thymidylate synthase inhibitor, a topoisomerase inhibitor and combinations thereof. Preferably, the alkylating agent preventing replication is oxaliplatin, the fluoropyrimidine is 5-fluorouracil, UFT, Utefos or Capecitabine, the thymidylate synthase inhibitor is raltitrexed, and the topoisomerase I inhibitor is irinotecan.

In the present invention, "antitumor agent" is understood as that physical or biological chemical agent or compound with antiproliferative, antioncogenic and/or carcinostatic properties that can be used for inhibiting the growth, proliferation and/or development of tumors. Examples of antitumor agents that can be used in the present invention are (i) antimetabolites, such as antifolates and purine analogues; (ii) natural products, such as antitumor antibiotics and mitotic inhibitors; (iii) hormones and antagonist thereof, such as androgens and corticosteroids; and (iv) biological agents, such as viral vectors. A list of compounds that can be used as antitumor agents is described in patent application WO2005/112973

In a particular embodiment of the invention, the antitumor agent comprises antiangiogenic agents and signaling pathway inhibitors which, in another more particular embodiment, the antiangiogenic agent is bevacizumab and the signaling pathway inhibitor is cetuximab, panitumumab, or erlotinib.

Putting the method of the invention into practice comprises obtaining a biological sample from the individual to be studied. Illustrative, non-limiting examples of said samples include different types of biological fluids, such as blood, serum, plasma, cerebrospinal fluid, feces, urine and saliva, as well as tissue samples. The samples of biological fluids can be obtained by any conventional method as can the tissue samples; by way of illustration, said tissue samples can be biopsy samples obtained by surgical resection.

Therefore, in a particular embodiment, the biological sample is a tissue sample or a biological fluid which, in yet another more particular embodiment is a tumor tissue sample.

The method of the invention can be applied to any stage of colorectal adenocarcinoma, from stage I to IV. Nevertheless, in a particular embodiment, the colorectal adenocarcinoma is large intestine adenocarcinoma in stage II or III or IV.

In another aspect, the invention relates to a reagent capable of detecting the expression levels of the genes of Tables 1 and 2 or the proteins encoded by said genes.

In a particular embodiment, the reagent comprises
(i) a set of nucleic acids, wherein each nucleic acid is able to hybridize specifically with one of the genes of Tables 1 and 2 or with the products of their transcription, or
(ii) a set of antibodies, or a fragment thereof able to detect an antigen, wherein each antibody or fragment is able to bind specifically to one of the proteins encoded by the genes of Tables 1 and 2.

In another more particular embodiment, the nucleic acids are DNA, cDNA or RNA probes and/or primers.

In another aspect, the invention relates to a DNA or RNA array comprising a set of nucleic acids, wherein said set of nucleic acids consists of the nucleic acid sequences of the genes of Tables 1 and 2, or a fragment thereof, or the products of their transcription.

In a particular embodiment, the DNA or RNA array of the invention furthermore comprises a nucleic acid molecule of one or several constitutively expressed genes.

Finally, in another aspect the invention also relates to an array comprising a set of antibodies, wherein said set of antibodies consists of antibodies, or fragments thereof, which are able to bind specifically with the proteins encoded by the genes of Tables 1 and 2 or any functionally equivalent variant of said proteins.

In a particular embodiment, the array comprising a set of antibodies furthermore comprises antibodies, or fragments thereof, which are able to bind specifically with the proteins encoded by one or several constitutively expressed genes.

The following Example illustrates the invention and must not be interpreted as limiting of the scope thereof.

### EXAMPLE 1

Analysis of the dynamic change in the transcriptome after chemoradiotherapy

### I. MATERIAL AND METHODS

### Patients

The initial screening of changes in the transcriptome following CRT was performed on 25 patients who were subjected to a trial with preoperative CRT, after which fresh tumor samples were taken. The chemotherapy consisted of four preoperative and postoperative cycles of oxaliplatin and raltitrexed. Only 6 of the 25 patients were selected for the dynamic analysis by means of serial analysis of gene expression (SAGE), from whom only 3 suitable SAGE libraries were obtained.

Patient A presented an adverse progress, paradigmatic of resistance to CRT. The other two patients, B and C, presented tumors representative of the tumor regression grades (TRG) II-III, the most common. Patient A started with a rectal adenocarcinoma in a favorable clinical stage (uT2N0), adjacent to the anal margin, which required abdominoperineal resection. Tumor substaging was not obtained, the TRG was 2, and the distant and local recurrences were recorded at 16 and 48 months respectively.

Patients B and C presented disease with a clinical stage uT3N0 at the time of the diagnosis, the first disease-free patient being found at the end of five years, and the second one with pulmonary metastases 54 months after starting the treatment.

For the study of multiplex quantitative PCR (qRT-PCR), 129 additional patients with stages II-III of rectal adenocarcinoma under 10 centimeters from the anal margin, who received two or more chemotherapy cycles and 50.4 Gy of radiotherapy before surgery, were recruited prospectively and the clinical-pathological variables were recorded (Table 3).

### Table 3: Patients and tumor characteristics

**Table 1.**

| **Patients and tumor characteristics** | **Total** | | **TRG 0/1** | | **TRG 2/3** | | **TRG 4** | |
|---|---|---|---|---|---|---|---|---|
| | | | **NTR** | | | **TR** | | |
| | **n** | **%** | **n** | **%** | **n** | **%** | **n** | **%** |
| **Total number of patients Mean age (range)** | 94 | 100 | 17 | 18.1 | 64 | 68.1 | 13 | 13.8 |
| | 63(23 | | 65 (37- | | 64 (23- | | 63 (45- | |
| | -84) | | 75) | | 84) | | 73) | |
| **Sex** | | | | | | | | |
| Men | 54 | 57.4 | 10 | 58.8 | 37 | 57.8 | 7 | 53.8 |
| Women | 40 | 42.6 | 7 | 41.2 | 27 | 42.2 | 6 | 46.2 |
| **Anal distance** | | | | | | | | |
| <6 cm | 57 | 60.6 | 12 | 70.6 | 41 | 64.1 | 4 | 30.8 |
| >6 cm | 37 | 39.4 | 5 | 29.4 | 23 | 35.9 | 9 | 69.2 |
| **Post-CRT** | | | | | | | | |
| **pathological** | | | | | | | | |
| **tumor** | | | | | | | | |
| **classification** | | | | | | | | |
| **(yp ^{T})** | | | | | | | | |
| ypT0 | 15 | 15.9 | 0 | 0 | 3 | 4.7 | 12 | 92.3 |
| ypT1 | 4 | 4.2 | 1 | 5.9 | 3 | 4.7 | - | - |
| ypT2 | 21 | 22.4 | 4 | 23.5 | 17 | 26.6 | - | - |
| ypT3 | 52 | 55.4 | 11 | 64.7 | 40 | 62.5 | 1 | 7.7 |
| ypT4 | 2 | 2.1 | 1 | 5.9 | 1 | 1.5 | - | - |
| **Post-CRT pathological node classification (yp N)** | | | | | | | | |
| ypN0 | 76 | 80.9 | 11 | 64,7 | 52 | 81.3 | 13 | 100 |
| ypN1 | 12 | 12,9 | 3 | 17.6 | 9 | 14.2 | 0 | 0 |
| ypN2 | 6 | 6.5 | 3 | 17.7 | 3 | 4.7 | 0 | 0 |
| **Preoperative CRT** | | | | | | | | |
| Oxaliplatin-Raltitrexed | 49 | 52.1 | 5 | 29.4 | 37 | 57.8 | 7 | 53.8 |
| 5-FU in infusion/UFT | 45 | 47.9 | 12 | 70.6 | 27 | 42.2 | 6 | 46.2 |
| **Recurrence patterns** | | | | | | | | |
| Local | 6 | 21.4 | 2 | 22.2 | 4 | 20 | 0 | 0 |
| Distant | 20 | 71.4 | 6 | 66.6 | 15 | 75 | 1 | 100 |
| Both | 2 | 7.2 | 1 | 11.2 | 1 | 5 | 0 | 0 |
| **Death** | | | | | | | | |
| Yes | 14 | 14.9 | 4 | 23.5 | 10 | 15.6 | 0 | 0 |
| No | 80 | 85.1 | 13 | 76.5 | 54 | 84.4 | 13 | 100 |

| | | | | | | | | |
|---|---|---|---|---|---|---|---|---|
| CRT: Chemoradiotherapy; TR: Tumor response; NTR: No tumor response; TRG: Tumor regression grade | | | | | | | | |

94 patients were included in the statistical analysis, 34 of whom were discarded as a result of incomplete gene expression data or inappropriate pathological samples.

The dynamic evaluation of the changes in the gene expression was analyzed in tumors from 74 patients, 21 of which were rejected because they were cases of complete pathological response or of lacking sufficient tumor material for the analysis.

In all the cases, informed consents were obtained, and the study was previously approved by the corresponding hospital ethics committee.

### Follow up

Follow-up was performed on the patients at three-month intervals for two years, after that every six months for three years and after that, yearly. The evaluations consisted of a physical examination, hemogram and biochemical study. Colonoscopy, echography and computed tomography were performed according to standard guidelines (http://www.asco.org/asco/downloads/Colon_Cancer_Tool_11-1-05.x1s.).

Whenever possible, a histopathological confirmation of recurrence was recommended.

### Response and tumor regression grade (TRG)

The TRG was determined by means of the histopathological examination of the surgical specimens, according to the amount of viable tumor cells and fibrosis, according to the system proposed by Dworak (Dworak, O. et al., 1997 Int. J Colorectal Dis., 12: 19-23). The response to CRT was classified in two groups based on the TRG, no tumor response (NTR) and tumor response (TR), given that patients with TRG 0/1 clearly present worse survival than the rest of the TRGs (Rodel, C. 2005, et al. J Clin Oncol, 23:8688-8696). NTR included TRG 0 and 1 (no regression or less than 25 % of the tumor mass); TR encompassed TRG 2 and 3 (regression greater than 25% of the tumor mass with at least some viable tumor cells) and TRG 4 (complete pathological response, no viable tumor cells).

### Preparation of the samples

For the SAGE analysis, fresh tumor samples were taken from the biopsies prior to and after CRT in the six patients selected. The tumor biopsies were frozen for 15 minutes with liquid nitrogen at -70°C. To isolate the RNA, the tumor samples stained with hematoxylin and eosin were examined by a pathologist; the frozen samples from the endoscopic biopsies were subjected to five micron serial cuts in their entirety; 20 sections with the same thickness were made in the frozen surgical specimens. It was required that all the samples contained at least 90% of tumor samples after the microdissection.

Out of the previously mentioned 129 recruited patients, 94 with complete clinical-pathological information and sufficient tumor sample for the analysis with low density arrays by means of multiplex quantitative PCR were selected. The paraffin-embedded samples were stained with hematoxylin-eosin and analyzed by a pathologist. Tumor cell enrichment greater than 75% was required, and where necessary, a subsequent macrodissection with a safety knife and a new hematoxylin-eosin staining in the postoperative biopsies were performed. The RNA was extracted from 5-10 five micron sections of the paraffined samples.

### Libraries and SAGE analysis

Six SAGE libraries were generated with the tumor samples from the endoscopic biopsies upon diagnosis and from the posttreatment surgical specimens from the three patients selected. The sections were directly homogenized and the resulting lysate was used directly for the modified SAGE methodology (microSAGE), following the previously described protocol (Datson, 1300-7) with minor modifications. A step of heating at 65°C for 10 minutes followed by two minutes in ice to allow a better separation of the concatemers was introduced. The products larger than 400 bp and smaller than 1,000 bp were cleaved, extracted and cloned in the Sphl site of the pZerO-1 vector (Invitrogen). The sequence files were analyzed using the SAGE 2000 program (kindly supplied by Kenneth W. Kinzler, Johns Hopkins University), and the resources of the webpage of the National Center for Biotechnology SAGE (http://www.ncbi.nlm.nih.gov/SAGE). Approximately 60,000 tags for the whole project, about 10,000 tags per SAGE library, were sequenced. To compare these SAGE libraries, each tag number was normalized with the SAGE 2000 program. The analysis of statistical significance was performed using the Monte Carlo analysis, which allows performing a large number of comparisons based on the results of the SAGE. The mapping between tags and genes was performed according to the SAGE Genie database, with reference to SAGEmap.

The genes which were regulated significantly after CRTs were selected for the subsequent exploratory phase in a wide patient series by means of multiplex quantitative PCR. This selection was carried out based on the statistically significant changes of the expression according to the Monte Carlo analysis (p<0.05), the similar tendency of the regulation before and following treatment (sub- or overregulation) in three patients, and finally the biological plausibility according to the earlier available literature.

### Multiplex quantitative PCR

The entire RNA content was measured and verified spectrophotometrically and electrophotometrically. The reverse transcription was performed based on 200 ng of total RNA using the High-Capacity cDNA Archive Kit (Applied Biosystems, Foster City, CA). The multiplex quantitative PCR reactions were performed in 384-well plates by means of the ABI PRISM 7900 HT Sequence Detection System (Applied Biosystems) in 100 µl samples with TaqMan Universal PCR Master Mix (Applied Biosystems) and 100 µl of cDNA corresponding to 50 ng of total RNA per channel of microfluidic card. The expression of each gene was measured in triplicate, and normalized in relation to three reference genes (GAPDH, B2M, and PMSB4). The explored genes included 25 obtained from the screening by means of SAGE (Table 4), and the rest from the literature (Table 5).

**Table 4. Blind screening of changes in the transcriptome after CRT by means of SAGE**

| **Gene** | **Patient A Pre/post (P)** | **Patient B Pre/Post (P)** | **Patient C Pre/post (P)** | **Unigene ID** | **Description** |
|---|---|---|---|---|---|
| MYO5B | 57/23 | 54/43 | 92/23 | 550481 | Myosin VB |
| | =0 | 0.11 | =0 | | |
| XIST | 4/11 | 3/12 | 4/11 | 529901 | X (inactive)-specific traffic |
| | 0.0665 | 0.02 | 0.056 | | |
| S100A10 | 8/13 | 13/4 | 2/8 | 143873 | S100 calcium binding protein A10 |
| | 0.21739 | 0.01944 | P=0.0457 | | |
| K-ALPHA- 1 | 4/9 | 3/10 | 3/11 | 524390 | Tubulin, alpha, ubiquitous |
| | 0.1323 | 0.0529 | 0.02292 | | |
| HLA-C | 12/15 | 7/17 | 6/22 | 534125 | Major histocompatibility complex, class I, C |
| | 0.4310 | 0.0397 | 0.00118 | | |
| COL1A1 | 5/37 | 10/34 | 18/39 | 172928 | Collagen, type I, alpha |
| | -0 | 0.00030 | 0.00245 | | |
| TFF3 | 26/10 | 15/15 | 21/5 | 82961 | Trefoil factor 3 (intestinal) |
| | 0.00456 | 0.7 | 0.00173 | | |
| FXYD3 | 86/36 | 52/16 | 4/22 | 301350 | FXYD domain contains iron transport regulator 3 |
| | =0 | =0 | 0.00012 | | |
| CKB | 40/14 | 25/11 | 10/14 | 173724 | Creatinine kinase, brain |
| | 0.00025 | 0.0101 | 0.23 | | |
| CCNB1IP1 | 123/99 | 104/79 | 81/100 | 107003 | Cyclin B1 interacting protein 1 |
| | 0.04737 | 0.0266 | 0.05148 | | |
| HIG2 | 13/14 | 17/7 | 9/7 | 521171 | Hypoxia-inducing protein 2 |
| | 0.5348 | 0.02536 | 0.44 | | |
| PH-4 | 0/5 | 0/5 | 0/3 | 271224 | Factor prolyl-4-hydroxylase Hypoxia-inducing protein 2 |
| | 0.032765 | 0.03384 | 0.1165 | | |
| CHC1 | 7/1 | 6/3 | 2/1 | 469723 | Chromosome condensation 1 |
| | 0.032457 | 0.22 | 0.5 | | |
| BAT1 | 7/1 | 4/1 | 3/2 | 254042 | HLA-B associated transcript 1 |
| | 0.032457 | 0.1675 | 0.5122 | | |
| SELENBP1 | 15/5 | 14/5 | 20/6 | 334841 | Selenium binding protein 1 |
| | 0.018875 | 0.0304 | 0.0068 | | |
| ANGPTL4 | 9/4 | 5/6 | 12/4 | 9613 | Angiopoietin-like 4 |
| | 0.11 | 0.5 | 0.052 | | |
| SMAD2 | 258/185 | 266/178 | 297/184 | 465061 | SMAD, mothers against DPP homolog 2 Drosophila) |
| | 0.00013 | =0 | 0.00001 | | |
| ZYX | 8/23 | 9/13 | 6/16 | 490415 | Zyxin |
| | 0.006 | 0.3 | 0.022 | | |
| GPX2 | 71/23 | 46/17 | 13/16 | 2704 | Glutathione peroxidase 2 (gastrointestinal) |
| | =0 | =0 | 0.3381 | | |
| MMP14 | 4/10 | 2/12 | 3/15 | 2399 | Matrix 14 metalloproteinase (inserted-membrane) |
| | 0.0815 | 0.006 | 0.00311 | | |
| ITGB4 | 0/6 | 1/5 | ¾ | 370255 | Integrin, beta 4 |
| | 0.017 | 0.1035 | 0.48 | | |
| WARS | 9/2 | 9/1 | 5/3 | 497599 | Tryptophanyl-tRNA synthetase |
| | 0.0354 | 0.0091 | 0.36992 | | |
| PCYT2 | 41/20 | 23/8 | 3/11 | 514635 | Phosphate cytidylyltransferase 2, ethanolamine |
| | 0.0039 | 0.0045 | 0.02292 | | |
| GSTP1 | 13/10 | 16/7 | 8/12 | 523836 | Glutathione S-transferase pi |
| | 0.324 | 0.0458 | 0.20702 | | |
| EEF2 | 52/34 | 45/32 | 40/37 | 515070 | Eukaryotic translation elongation factors |
| | 0.0257 | 0.0754 | 0.48 | | |

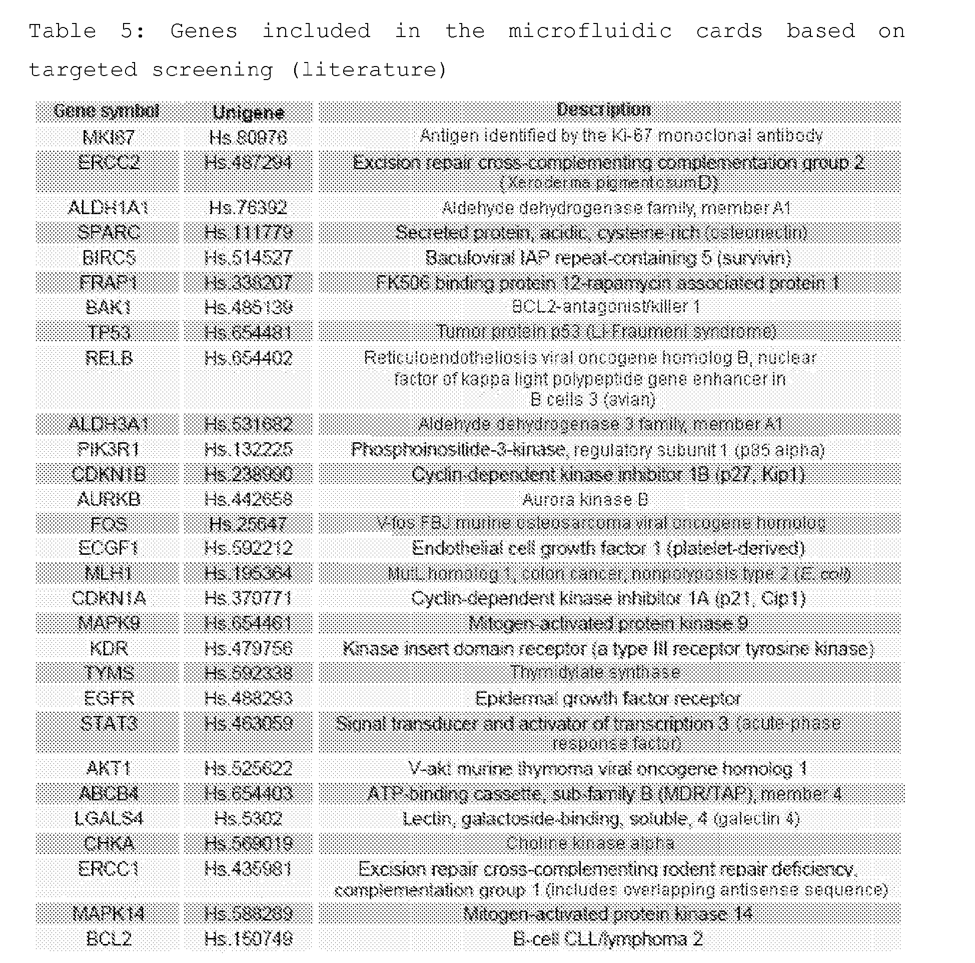

### Statistical analysis

*Descriptive statistics.* The absolute frequencies of onset of each modality of the variable and the relative frequencies expressed in percentages were calculated in the case of qualitative variables. The measurements of median or mean central tendency were calculated for the quantitative variables. The standard deviation and the range of the variable were used as measures of dispersion.

*Inferential statistics.* The chi square test with Yates' correction was applied for qualitative variables and Fisher's exact test was applied in the case of 2x2 Tables. Before applying the hypothesis test for quantitative variables, the Kolmogorov-Smirnov (KS) test was applied to study the type of distribution the variable followed (normal, non-normal distribution). For those variables in which the KS test indicated that it followed a normal distribution, parametric tests (Student's t), and in the other case non-parametric tests (Wilcoxon, Mann-Whitney U), were applied. As one of the objectives of the study is the assessment of the change in the expressions of genes before and after treatment with chemotherapy, part of the tests applied corresponded with tests for paired or dependent samples. The Kaplan-Meier method was used to estimate the overall survival.

### Normalization of Cₜ·

Data from the PCR-QT were normalized as a step prior to the analysis thereof. Two models were used for that purpose, which models were subsequently evaluated; a) normalizing with three genes and b) normalizing with 12 genes. The first method was applied due to the variability obtained with the type b) normalization. In all the cases it was normalized by the mean of each gene calculated in the entire patient series.

### Correlation of the dynamic changes in the data from qRT-PCR with prognosis.

The Wilcoxon test was used to study the changes in the expression of the genes analyzed after chemotherapy. Empirical p values for each gene were obtained through 5,000 permutation tests. The genes with statistically significant differences in the expression (p<0.01) were selected for discriminant and Cox's proportional hazards regression analysis. Cox's multivariate proportional hazards regression analysis (adjusted for sex, stage and number of positive ganglions) was performed for each gene in the training set (60% of the sample, the remaining 40% was the testing set). The assumption of proportional hazards for the covariables was investigated by examining the Schoenfeld residuals.

### II. RESULTS

### Clinical and pathological data

The histopathological and gene expression data were obtained in 94 patients for the multiplex quantitative PCR analysis. They are illustrated in Table 3.

### TRG as a prognostic factor

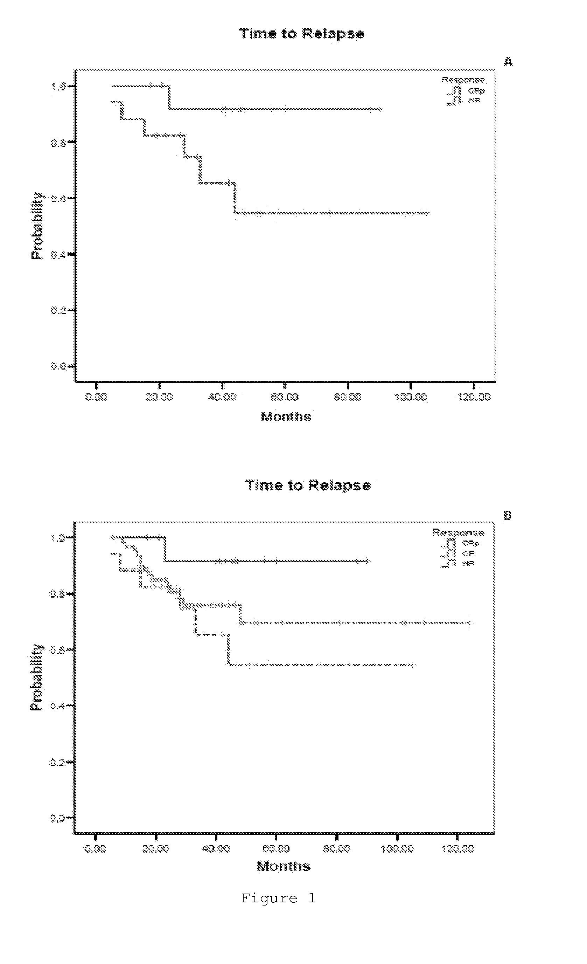

TRGs 0/1, 2/3, and 4 were found in 18.5%, 67.4%, and 14.1% of the resected tumors, respectively. The TRG was related to the disease-free survival (DFS) of the patients, a clear tendency to relapse at a lower TRG being observed. The analysis of the end groups (TRG 0/1 versus TRG 4) showed statistically significant differences in the DFS (p=0.05, Figure 1).

### Screening of changes in the transcriptome after CRT by means of SAGE

Six libraries of genes in patients A, B and C were generated. The comparison of evolutionary changes in the transcriptome before and after treatment, through the Monte Carlo analysis, showed those which were significantly regulated. According to this analysis, a collection of genes with changes in their expression common in the three patients, in the same positive or negative regulation sense, the biological plausibility furthermore being considered, was selected. 17 genes were unidirectionally regulated showing p <0.05 in at least two of the patients, and statistical significance was achieved in one of the patients in seven genes. 25 genes were thus selected for their subsequent evaluation in a wide patient series (Table 4).

### Multiplex quantitative PCR and tumor response

The supervised analysis considering the type of member response (TR) versus no response (NTR) and the basal gene expression data, in the sample of 94 patients, determined a profile of 13 genes able to correctly classify 96.5% of the patients who experience TR (sensitivity), and 80% of the cases with NTR; this genomic imprinting offers a precision of 93.1% of the patients (Table 6).

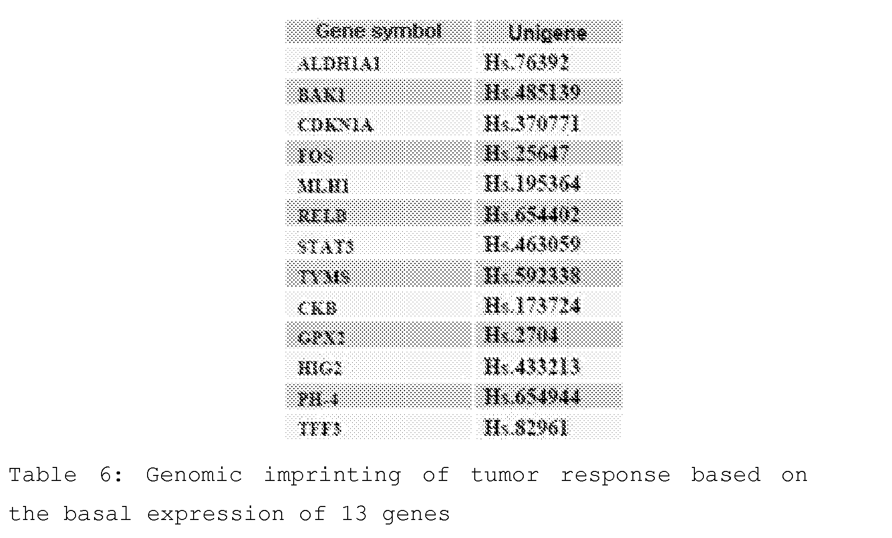

### III. Discussion

A profile of 13 genes was identified, which profile validated in a part of the sample, confirms that it correctly classifies the response of 93.1% of patients. The sensitivity for response is greater than 96%, conclusively indicating which patients will respond to said treatment and, therefore, in whom it is of great interest to apply this treatment. The patients who will benefit the most from this prediction are those in whom there is not a clear indication of neoadjuvant chemotherapy, in virtue of the clinical uncertainties (patients with intermediate risk, such as cases of T2N1, T3N0 disease; those with deficient clinical staging, especially frequent in relation to nodal staging, elderly patients with a doubtful risk to benefit ratio, etc.). The no response is predicted with an elevated sensitivity of 80%. Again this prediction is of great interest, since given the very low probability of benefit, these patients must depart from the usual preoperative treatment protocol and be operated on directly, preventing unnecessary delays in the local treatment, which could give rise to these patients having a greater risk of developing distant metastasis. Additionally, exposure to the considerable toxicity of chemoradiotherapy would be avoided in these patients.

The results on the classification of the tumor response are not directly comparable with the Ghadimi study, since in the study herein described it is considered more sensitive and objective to measure the tumor response grade exclusively through the TRG, since upon considering substaging as a marker, in addition to being based on rather imprecise clinical tests, the histological responses which do not alter staging and which seem to involve a prognosis equally favorable to other responses go unnoticed. Despite this fact, sensitivity and precision were greater in the present invention (96.5% versus 78% and 93.1 % versus 83%) with a much lower number of genes required.

In addition, in the present invention it is the first time that it is shown that a gene profile is able to predict the tumor regression grade in rectal adenocarcinomas from paraffined samples. This aspect is important for the clinical application, since the collection of frozen samples is not a common task in the routine clinical practice.

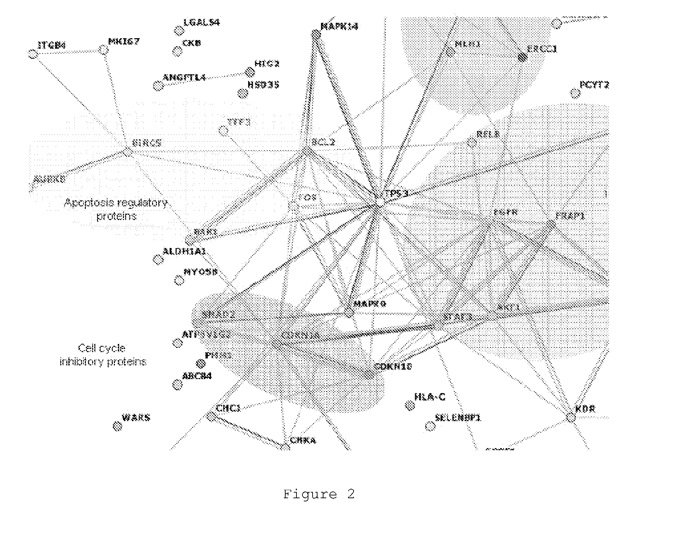

As is shown in Figure 2, the proteins corresponding to the genes making up the genomic imprint form a lattice in which protein p53 occupies a central position where different pathways converge. p53 plays a fundamental pleiotropic function to maintain the integrity of the cell DNA and to that end, it acts in different levels. When damage occurs in the DNA, p53 activates signaling pathways which cause cell cycle arrest, activating the transcription of cell cycle inhibiting genes such as CDKN1A (Cdk inhibitor p21 protein) and also negatively regulating cell proliferation pathways. Once the cycle is arrested, it activates DNA repair genes such as ERCC1, ERCC2 and MLH1. If the damage is severe and cannot be repaired, p53 activates apoptosis pathways, negatively regulating antiapoptotic genes such as Bc12 or BIRC5, and positively regulating proapoptotic genes such as Bak1.

## Claims

1. An *in vitro* method for predicting the clinical response of an individual diagnosed with colorectal adenocarcinoma to a therapy comprising
(i) quantifying the expression levels of the genes shown in Tables 1A and 1B, or
(ii)quantifying the levels of proteins encoded by the genes of Tables 1A and 1B, or any functionally equivalent variant of said proteins,
in a biological sample from said individual before the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of a better response of the individual after the administration of the therapy.

2. An *in vitro* method for predicting the clinical response of an individual diagnosed with colorectal adenocarcinoma to the therapy, comprising
(i) quantifying the expression levels of the genes shown in Tables 2A and 2B, or
(ii)quantifying the levels of proteins encoded by the genes of Tables 2A and 2B, or any functionally equivalent variant of said proteins,
in a biological sample from said individual before the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of worse response of the individual after the administration of the therapy.

3. Method according to claims 1 and 2, wherein the parameter indicating the progress of an individual diagnosed with colorectal adenocarcinoma before the administration of the therapy is selected from the group of tumor response, risk of relapse, disease-free survival and/or overall survival of the individual.

4. An *in vitro* method for monitoring the effect of the therapy administered to an individual diagnosed with colorectal adenocarcinoma, comprising
(i) quantifying the expression levels of the genes shown in Tables 1A and 1B, or
(ii)quantifying the levels of proteins encoded by the genes of Tables 1A and 1B, or any functionally equivalent variant of said proteins,
in a biological sample from said individual after the administration of the therapy, wherein an elevated expression of the genes of Table 1A or of the expression of the protein corresponding to each gene, or any functionally equivalent variant of said protein, and a reduced expression of the genes of Table 1B or of the expression of the protein corresponding to each gene, or any functionally equivalent variant of said protein, with respect to genes which are constitutively expressed, or their corresponding proteins, is indicative of good tumor response to the therapy.

5. An *in vitro* method for monitoring the effect of the therapy administered to an individual diagnosed with colorectal adenocarcinoma, comprising
(i) quantifying the expression levels of the genes shown in Tables 2A and 2B, or
(ii)quantifying the levels of proteins encoded by the genes of Tables 2A and 2B, or any functionally equivalent variant of said proteins,
in a biological sample from said individual after the administration of the therapy, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative of poor tumor response to the therapy.

6. An *in vitro* method for designing a customized therapy for an individual diagnosed with colorectal adenocarcinoma, comprising
(i) quantifying the expression levels of the genes shown in Tables 2A and 2B, or
(ii)quantifying the levels of proteins encoded by the genes of Tables 2A and 2B, or any functionally equivalent variant of said proteins,
in a biological sample from said individual, wherein an elevated expression of the genes of Table 2A or of the expression of the protein corresponding to each gene, or any functionally equivalent variant of said protein, and a reduced expression of the genes of Table 2B or of the expression of the protein corresponding to each gene, or any functionally equivalent variant of said protein, with respect to genes which are constitutively expressed, or their corresponding proteins, is indicative that the individual needs an alternative therapy with respect to standard therapy.

7. Method according to any of claims 1 to 6, wherein quantifying the expression levels of the genes of Tables 1 and 2 comprises quantifying the messenger RNA (mRNA) of the genes of Tables 1 and 2, or a fragment of said mRNA, the complementary DNA (cDNA) of the genes of said Tables 1 and 2, or a fragment of said cDNA, or mixtures thereof.

8. Method according to any of claims 1 to 7, wherein the expression levels of the genes of Tables 1 and 2 are quantified by means of a multiplex quantitative polymerase chain reaction (PCR) or a DNA or RNA array.

9. Method according to any of claims 1 to 8, wherein quantifying the levels of the protein encoded by the genes of Tables 1 and 2 comprises quantifying the proteins encoded by the genes of said Tables 1 and 2.

10. Method according to any of claims 1 to 9, wherein the levels of protein encoded by the genes of Tables 1 and 2 are quantified by means of Western blot, ELISA or a protein array.

11. Method according to any of claims 1 to 10, wherein the therapy is a cytotoxic and/or cytostatic treatment.

12. Method according to claim 11, wherein the cytotoxic and/or cytostatic treatment is chemotherapy, radiotherapy, antitumor agents or combinations thereof.

13. Method according to claim 12, wherein the chemotherapy comprises a compound selected from an alkylating agent preventing replication, a fluoropyrimidine, a thymidylate synthase inhibitor, a topoisomerase inhibitor and combinations thereof.

14. Method according to claim 12, wherein the antitumor agents comprise antiangiogenic agents and signaling pathway inhibitors.

15. Method according to claim 14, wherein the antiangiogenic agent is bevacizumab and the signaling pathway inhibitor is cetuximab, panitumumab, or erlotinib.

16. Method according to claim 13, wherein the alkylating agent preventing replication is oxaliplatin, the fluoropyrimidine is 5-fluorouracil, UFT, Utefos or capecitabine, the thymidylate synthase inhibitor is raltitrexed and the topoisomerase I inhibitor is irinotecan.

17. Method according to any of claims 1 to 16, wherein the biological sample is a tissue sample or a biological fluid.

18. Method according to claim 17, wherein the tissue sample is tumor tissue sample.

19. Method according to any of claims 1 to 18 wherein the colorectal cancer is colorectal adenocarcinoma in stages II or III or IV.

20. A reagent capable of detecting the expression levels of the genes of Tables 1 and 2 or the proteins encoded by said genes.

21. Reagent according to claim 23, wherein the reagent comprises
(i) a set of nucleic acids, wherein each nucleic acid is able to hybridize specifically with one of the genes of Tables 1 and 2 or with the products of their transcription, or
(ii) a set of antibodies, or a fragment thereof able to detect an antigen, wherein each antibody or fragment is able to bind specifically to one of the proteins encoded by the genes of Tables 1 and 2 or to any functionally equivalent variant of said protein.

22. Reagent according to claim 24, wherein the nucleic acids are DNA, cDNA or RNA probes and/or primers.

23. A DNA or RNA array comprising a set of nucleic acids, wherein said set of nucleic acids consists of the nucleic acid sequences of the genes of Tables 1 and 2, or a fragment thereof, or the products of their transcription.

24. Array according to claim 23, further comprising a nucleic acid molecule of one or several constitutively expressed genes.

25. An array comprising a set of antibodies, wherein said set of antibodies consists of antibodies, or fragments thereof, which are able to bind specifically with the proteins encoded by the genes of Tables 1 and 2 or any functionally equivalent variant of said proteins.

26. Array according to claim 25, further comprising antibodies, or fragments thereof, which are able to bind specifically with the proteins encoded by one or several constitutively expressed genes.
